(19) Europäisches Patentamt
European Patent Office
Office européen des brevets

(11) Veröffentlichungsnummer: **0 080 675**
**B1**

# (12) EUROPÄISCHE PATENTSCHRIFT

(45) Veröffentlichungstag der Patentschrift: **20.03.85**

(51) Int. Cl.⁴: **C 07 J 19/00**, A 61 K 31/705

(21) Anmeldenummer: **82110729.9**

(22) Anmeldetag: **20.11.82**

(54) **Neue Ketale von 3'''-Dehydro-cardenolid-tridigitoxosiden, Verfahren zu ihrer Herstellung und ihre Verwendung als Arzneimittel.**

(30) Priorität: **26.11.81 DE 3146899**

(43) Veröffentlichungstag der Anmeldung: **08.06.83 Patentblatt 83/23**

(45) Bekanntmachung des Hinweises auf die Patenterteilung: **20.03.85 Patentblatt 85/12**

(84) Benannte Vertragsstaaten: **AT BE CH DE FR GB IT LI LU NL SE**

(56) Entgegenhaltungen:
**BE-A-752 284**
**FR-A-2 081 328**

(73) Patentinhaber: **Boehringer Mannheim GmbH, *Sandhoferstrasse 116, D-6800 Mannheim 31 (DE)***

(72) Erfinder: **Schaumann, Wolfgang, Prof. Dr. med., Mönchhofstrasse 58, D-6900 Heidelberg (DE)**
Erfinder: **Kaiser, Fritz, Dr. rer. nat., Hans-Holbein-Strasse 20, D-6840 Lampertheim (DE)**
Erfinder: **Voigtländer, Wolfgang, Dr. rer. nat., Haselnussweg 30, D-6940 Weinheim (DE)**
Erfinder: **Hoyer, Edgar, In den alten Wiesen 55, D-6800 Mannheim (DE)**
Erfinder: **Neubert, Peter, Dr. rer. nat., Telemannstrasse 5, D-6940 Weinheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Gegenstand der Erfindung sind neue Verbindungen der Formel I

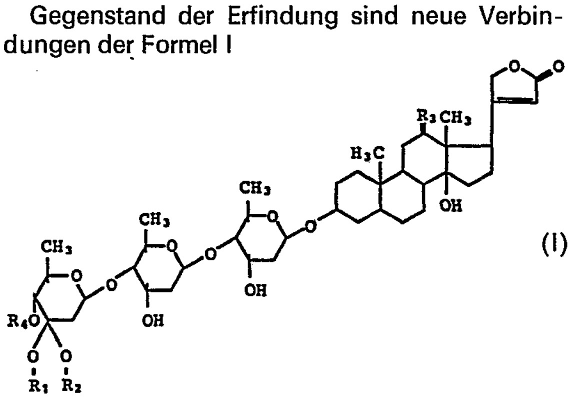

(I)

in der

$R_1$ und $R_2$ gleiche Alkylgruppen mit 1 bis 3 Kohlenstoffatomen oder gemeinsam ein cyclisches Ketal mit 2 bis 6 Kohlenstoffatomen,

$R_3$ 2 Wasserstoffatome, die Gruppe 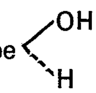 oder

den Rest 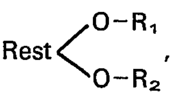, worin $R_1$ und $R_2$ die angegebene Bedeutung besitzen,

$R_4$ Wasserstoff, einen Alkanoylrest mit 1 bis 3 Kohlenstoffatomen oder eine Alkylgruppe mit 1 bis 3 Kohlenstoffatomen

darstellen

und Verfahren zu ihrer Herstellung sowie ihre Verwendung zur Herstellung von Arzneimitteln gegen Herzinsuffizienz.

Unter einem Alkanoylrest mit 1 bis 3 Kohlenstoffatomen ist vorzugsweise der Acetylrest zu verstehen.

Ein Alkylrest mit 1 bis 3 Kohlenstoffatomen ist vorzugsweise die Methylgruppe.

Die in der Therapie der Herzinsuffizienz hauptsächlich verwendeten Digitalisglykoside Digitoxin und Digoxin mit ihren Derivaten, z.B. Acetyldigoxin, Methyldigoxin, lassen für die Sicherheit ihrer Anwendung noch Wünsche offen: Digoxin und Derivate werden überwiegend durch die Nieren ausgeschieden und können deshalb bei Patienten mit verringerter Nierenfunktion zu Intoxikationen führen.

Digitoxin ist das Glykosid mit der längsten Verweildauer im Organismus, weshalb etwa auftretende Intoxikationen (z.B. bei Überdosierung) nur extrem langsam wieder abklingen können.

Es wurde nun gefunden, dass die erfindungsgemässen Ketale von 3'''-Dehydrocardenolidtridigitoxosiden eine ideale Mittelstellung einnehmen, indem sie überwiegend extrarenal ausgeschieden werden und somit bei verminderter Nierenfunktion weniger gefährlich sind und zudem erheblich schneller eliminiert werden als Digitoxin, und zwar Eliminationszeiten ähnlich der des Digoxins.

Die neuen Verbindungen der Formel I können wie folgt hergestellt werden:

Verbindung der Formel II

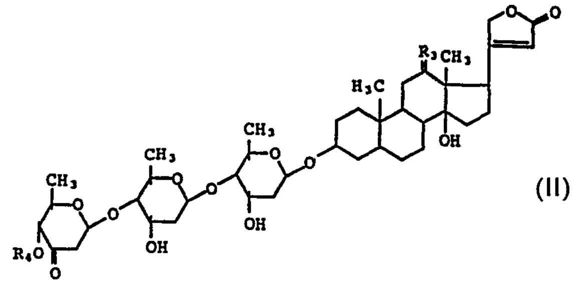

(II)

in der

$R_3$ 2 Wasserstoffatome, die Gruppe 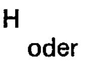 oder ein Sauerstoffatom,

$R_4$ Wasserstoff, einen Alkanoylrest mit 1 bis 3 Kohlenstoffatomen oder eine Alkylgruppe mit 1 bis 3 Kohlenstoffatomen bedeuten

werden nach an sich bekannten Methoden mit einwertigen Alkanolen (1 bis 3 Kohlenstoffatomen) oder zweiwertigen Alkanolen (2 bis 6 Kohlenstoffatomen) zu Ketalen umgesetzt.

Die Reaktion wird üblicherweise in dem betreffenden Alkanol als Lösungsmittel unter Zusatz von sauren Katalysatoren zwischen Raumtemperatur und dem Siedepunkt des Lösungsmittels durchgeführt. Durch wasserbindende Mittel, vorzugsweise Orthoameisensäureester oder ein unpolares Lösungsmittel, welches mit Wasser azeotrop destilliert, z.B. Benzol oder Toluol, wird die Reaktion gefördert.

Die Aufarbeitung der Reaktionsgemische und Reinigung der Endprodukte erfolgt nach üblichen Methoden u.a. unter Einsatz chromatographischer Verfahren oder durch multiplikative Verteilung und Kristallisation.

Identität und Reinheit der erhaltenen Verbindungen wurden durch Dünnschichtchromatogramme überprüft. Dabei wurden DC-Fertigplatten (Merck Kieselgel 60/F Imprägnierung 20% Formamid in Aceton) eingesetzt und mit Fliessmittel Heptan/Methylethylketon 1:1 + 1,8% Formamid bzw. Fliessmittel Xylol/Methylethylketon 2:3 + 5% Formamid im Beispiel 6 entwickelt. Die fertigen Chromatogramme wurden mit Trichloressigsäure/Chloramin-Reagens besprüht und die Substanzen durch ihre Fluoreszenzen im langwelligen UV ($\lambda$ = 360 nm) ermittelt. Die Laufstrecken (R) im Chromatogramm wurden jeweils auf einen mitgeführten Standard bezogen. Dabei bedeutet $R_{Dt}$ den auf die Laufstrecke von 3'''-Dehydrodigitoxin bezogenen R-Wert, $R_D$ den auf die Laufstrecke von 3''',12-Didehydrodigoxin bezogenen R-Wert und $R_{Dg}$ den auf die Laufstrecke von 3'''-Dehydrodigoxin bezogenen R-Wert.

Die erfindungsgemässen Cardenolidglykoside können in Einzeldosierungen von 0,05 bis 1,0 mg 1- bis 4mal täglich appliziert werden. Die Applikation erfolgt vorzugsweise oral, jedoch ist auch eine parenterale Applikation ohne weiteres möglich.

Als orale Darreichungsform werden bevorzugt Tabletten aber auch Steckkapseln und Weichgelatinekapseln verwendet. Zur individuellen Dosierungseinnahme z.B. für Kinder ist die Zubereitung als Liquidum geeignet. Für die Notfall- und Stationärbehandlung erfolgt die Anwendung durch Injektion entsprechender Lösungen.

Zur Herstellung von Tabletten oder Steckkapseln zur oralen Darreichung wird der Wirkstoff mit üblichen Hilfsstoffen, wie Lactose und Stärke homogen gemischt, wobei wegen der geringen Einzeldosierung die Herstellung einer Vormischung bevorzugt wird. Die Wirkstoff/Hilfsstoff-Mischung kann durch Auswahl geeigneter Hilfsstoffe als trockene Pulvermasse oder durch Granulation mit Bindemitteln, wie Stärkekleister oder Polyvinylpyrrolidon als Granulat in Steckkapseln abgefüllt oder nach weiterer Zumischung üblicher Sprengmittel und Gleitmittel zu Tabletten verpresst werden.

Trägerstoffe für Weichgelatinekapseln können übliche Glycerinfettsäureester sein, aber auch Polyethylenglykole als Lösungsmittel für den Wirkstoff. Für eine Liquidum- oder Ampullenform können als Lösungsmittel Ethanol oder mehrwertige Alkohole gegebenenfalls unter Zusatz von Wasser und anderen üblichen Hilfsstoffen verwendet werden.

Die Vorteile der erfindungsgemässen Verbindungen gegenüber Digoxin und Digitoxin, das heisst die Verbindung von rascher Ausscheidung mit hoher Ausscheidungsrate über Galle/Faeces ist in dem folgenden Versuchsprotokoll dargestellt.

*Versuchsprotokoll*

Je 4 Katzen erhielten je eine intravenöse Dosis von 20 µg/kg eines der in der folgenden Tabelle genannten Glykoside. Die Glykoside waren nach der Methode Haberland und Maerten* – Digoxin nach der Methode von Wartburg** – mit Tritium markiert.

In den nach 2 und 7 d getrennt gesammelten Urin- und Faeces-Portionen wurde die Radioaktivität bestimmt.

Die in der Tabelle zusammengestellten Werte geben die Ausscheidungsgeschwindigkeit (Spalte I) und den Anteil der Ausscheidung im Urin wieder (Spalte II).

Es bedeuten:

Spalte I: ausgeschiedene Menge in Harn + Faeces nach 2 d in Prozent der Gesamtausscheidung nach 7 d.

Spalte II: prozentualer Anteil der Ausscheidung im Harn nach 7 d bezogen auf die Gesamtausscheidung in Harn + Faeces nach 7 d.

| Glykosid | I | II |
|---|---|---|
| Digoxin | 53 | 44 |
| Digitoxin | 20 | 16 |
| 3'''-Dehydrodigitoxindimethylketal | 48 | 27 |
| 3'''-Dehydrodigitoxinethylenketal | 43 | 24 |

* DE-A Nr. 1959064
** „Biochem. Pharm.", *14*, 1883 (1965)

*Beispiel 1:*

*3'''-Dehydrodigitoxindimethylketal*

2 g 3'''-Dehydrodigitoxin in 40 ml Methylenchlorid (wasserfrei) und 40 ml Methanol gelöst, werden nach Zugabe von 10 ml Orthoameisensäuretriethylester und 50 mg p-Toluolsulfonsäure 1 d bei Raumtemperatur stehen gelassen, mit 80 ml 5% Natriumbicarbonatlösung verdünnt, mit Chloroform ausgeschüttelt, die Chloroformphasen mit Wasser gewaschen und im Vakuum eingeengt. Das Rohprodukt wird mit Cyclohexan/Essigester 2:1 über eine Cellulosesäule (mit Formamid imprägniert) getrennt. Die chromatographisch einheitlichen Fraktionen liefern nach Kristallisation aus Aceton 640 mg 3'''-Dehydrodigitoxindimethylketal.

Smp.: 206-210° C
$R_{Dt}$: 1,96

*Beispiel 2:*

*3'''-Dehydrodigitoxindiethylketal*

2 g 3'''-Dehydrodigitoxin in 40 ml Methylenchlorid und 40 ml Ethanol (absolut) gelöst, werden mit 10 ml Orthoameisensäuretriethylester und 50 mg p-Toluolsulfonsäure versetzt, 2 d bei Raumtemperatur stehen gelassen, wie unter Beispiel 1 beschrieben aufgearbeitet und das Rohprodukt mit Heptan/Methylethylketon 5:1 über eine Cellulosesäule (mit Formamid imprägniert) getrennt. Die chromatographisch einheitlichen Fraktionen ergeben nach Kristallisation aus Aceton 720 mg 3'''-Dehydrodigitoxindiethylketal.

Smp.: 189-193° C
$R_{Dt}$: 2,89

*Beispiel 3:*

*3'''-Dehydrodigitoxinethylenketal*

2 g 3'''-Dehydrodigitoxin in 40 ml Methylenchlorid und 40 ml Ethylenglykol gelöst, werden nach Zugabe von 10 ml Orthoameisensäuretriethylester und 50 mg p-Toluolsulfonsäure wie unter Beispiel 2 beschrieben umgesetzt und aufgearbeitet. Das Rohprodukt wird mit Tetrachlorkohlenstoff/Essigester 3:7 (+ 2% Wasser) über Silicagel (+ 2% Wasser) fraktioniert. Die chromatographisch einheitlichen Fraktionen liefern nach Kristallisation aus Aceton 550 mg 3'''-Dehydrodigitoxinethylenketal.

Smp.: 152-156° C
$R_{Dt}$: 1,26

*Beispiel 4:*

*3'''-Dehydrodigitoxinpropylenketal*

2 g 3'''-Dehydrodigitoxin in 40 ml Methylenchlorid und 40 ml 1,2-Propandiol gelöst, werden mit 10 ml Orthoameisensäuretriethylester und 50 mg p-Toluolsulfonsäure versetzt wie unter Beispiel 2 beschrieben umgesetzt und aufgearbeitet. Das Rohprodukt wird mit Cyclohexan/Essigester 2:1 über eine Cellulosesäule (mit Formamid imprägniert) getrennt. Die chromatographisch einheitlichen Fraktionen ergeben nach Kristallisation

aus Aceton 630 mg 3'''-Dehydrodigitoxinpropylenketal.

Smp.: 159-163°C

$R_{Dt}$: 1,68

*Beispiel 5:*

*3''',12-Didehydrodigoxinbisdiethylketal*

2 g 3''',12-Didehydrodigoxin in 40 ml Ethanol (wasserfrei) und 40 ml Methylenchlorid gelöst, werden nach Zugabe von 10 ml Orthoameisensäuretriethylester und 50 mg p-Toluolsulfonsäure 1 d bei Raumtemperatur stehen gelassen. Das Reaktionsgemisch wird mit 80 ml 5% Natriumbicarbonatlösung verdünnt, mit 6×30 ml Chloroform ausgeschüttelt, die Chloroformphasen mit Wasser gewaschen und im Vakuum eingeengt. Das Rohprodukt wird mit Cyclohexan und zunehmenden Mengen Essigester über Silicagel chromatographiert. Chromatographisch einheitliche Fraktionen liefern nach Kristallisation aus Aceton 0,6 g 3''',12-Didehydrodigoxinbisdiethylketal.

Smp.: 190-195°C

$R_D$: 6,0

*Beispiel 6:*

*3'''-Dehydrodigoxindimethylketal*

1 g 3'''-Dehydrodigoxin in 20 ml Methylenchlorid (wasserfrei) und 20 ml Methanol gelöst, wird nach Zugabe von 5 ml Orthoameisensäuretriethylester und 25 mg p-Toluolsulfonsäure wie unter Beispiel 1 beschrieben umgesetzt und aufgearbeitet. Das Rohprodukt wird mit Xylol/Methylethylketon 3:1 über eine Cellulosesäule (mit Formamid imprägniert) getrennt. Die chromatographisch einheitlichen Fraktionen liefern nach Kristallisation aus Aceton/Ether/Petrolether 410 mg 3'''-Dehydrodigoxindimethylketal.

Smp.: 131-135°C

$R_{Dg}$: 1,67

*Beispiel 7:*

*4'''-Acetyl-3'''-dehydrodigitoxinethylenketal*

1 g 3'''-Dehydrodigitoxinethylenketal in 10 ml Dimethylformamid gelöst, wird nach Zugabe von 230 mg Triethylendiamin und 0,20 ml Essigsäureanhydrid 24 h bei Raumtemperatur stehen gelassen, mit 80 ml Wasser verdünnt, mit Chloroform ausgeschüttelt und die Chloroformphasen im Vakuum eingeengt. Das Rohprodukt wird mit Heptan/Methylethylketon 3:1 über eine Cellulosesäule (mit Formamid imprägniert) getrennt. Die chromatographisch einheitlichen Fraktionen ergeben nach Kristallisation aus Aceton/Ether 510 mg 4'''-Acetyl-3'''-dehydrodigitoxinethylenketal.

Smp.: 239-243°C

$R_{Dt}$: 2,68

*Beispiel 8:*

*4'''-Methyl-3'''-dehydrodigitoxindiethylketal*

1 g 4'''-Methyl-3'''-dehydrodigitoxin in 20 ml Methylenchlorid und 20 ml Ethanol (absolut) gelöst, wird mit 5 ml Orthoameisensäuretriethylester und 25 mg p-Toluolsulfonsäure versetzt, 2 d bei Raumtemperatur stehen gelassen, wie unter Beispiel 1 beschrieben aufgearbeitet und das Rohprodukt mit Heptan/Methylethylketon 4:1 über eine Cellulosesäule (mit Formamid imprägniert) getrennt. Die chromatographisch einheitlichen Fraktionen liefern nach Kristallisation aus Ether/Petrolether 320 mg 4'''-Methyl-3'''-dehydrodigitoxindiethylketal.

Smp.: 226-230°C

$R_{Dt}$: 4,68

Das als Ausgangsmaterial verwendete 4'''-Methyl-3'''-dehydrodigitoxin ist neu und wird wie folgt hergestellt:

In ein Gemisch von 8 ml Pyridin und 150 ml Methylenchlorid werden unter Rühren bei Raumtemperatur 6 g Chromtrioxyd gegeben und 15 min bei Raumtemperatur gerührt. Eine Lösung von 8 g 4'''-Methyldigitoxin in 10 ml Pyridin und 100 ml Methylenchlorid wird langsam zugegeben, 15 min bei Raumtemperatur gerührt, 1 h unter Rückfluss zum Sieden erhitzt, mit 500 ml Wasser verdünnt, mit Chloroform ausgeschüttelt, die Chloroformphasen mit Natriumbicarbonatlösung (5%) und Wasser gewaschen, über Natriumsulfat getrocknet und im Vakuum eingeengt. Das Rohprodukt wird mit Heptan/Methylethylketon 2:1 über eine Cellulosesäule (mit Formamid imprägniert) getrennt. Die chromatographisch einheitlichen Fraktionen liefern nach Kristallisation aus Chloroform/Ether 4,2 g 4'''-Methyl-3'''-dehydrodigitoxin.

Smp.: 213-217°C

## Patentansprüche

1. Ketale von 3'''-Dehydrocardenolidtridigitoxosiden der Formel (I)

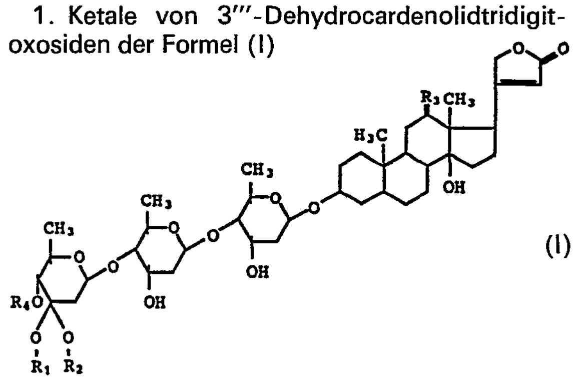

(I)

in der

$R_1$ und $R_2$ gleiche Alkylgruppen mit 1 bis 3 Kohlenstoffatomen oder gemeinsam ein cyclisches Ketal mit 2 bis 6 Kohlenstoffatomen,

$R_3$ 2 Wasserstoffatome, die Gruppe 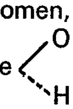 oder den Rest 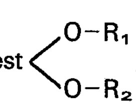, worin $R_1$ und $R_2$ die angegebene Bedeutung besitzen,

$R_4$ Wasserstoff, einen Alkanoylrest mit 1 bis 3 Kohlenstoffatomen oder eine Alkylgruppe mit 1 bis 3 Kohlenstoffatomen darstellen.

2. Verfahren zur Herstellung von Ketalen von 3'''-Dehydrocardenolidtridigitoxosiden der Formel (I)

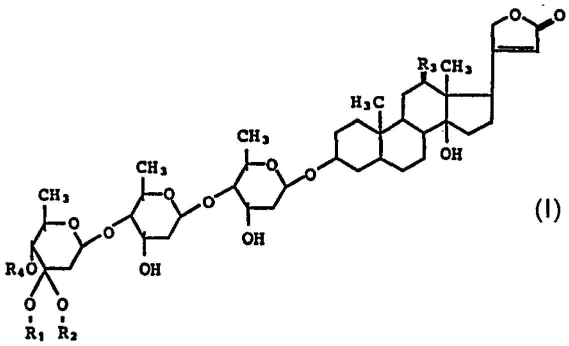

(I)

in der

$R_1$ und $R_2$ gleiche Alkylgruppen mit 1 bis 3 Kohlenstoffatomen oder gemeinsam ein cyclisches Ketal mit 2 bis 6 Kohlenstoffatomen,

$R_3$ 2 Wasserstoffatome, die Gruppe 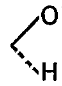 oder den Rest 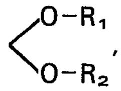, worin $R_1$ und $R_2$ die angegebene Bedeutung besitzen,

$R_4$ Wasserstoff, einen Alkanoylrest mit 1 bis 3 Kohlenstoffatomen oder eine Alkylgruppe mit 1 bis 3 Kohlenstoffatomen darstellen,

dadurch gekennzeichnet, dass man Verbindungen der Formel (II)

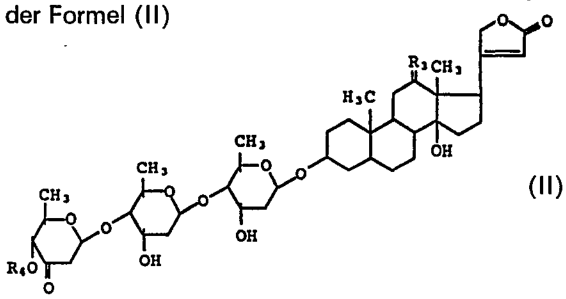

(II)

in der

$R_3$ 2 Wasserstoffatome, die Gruppe 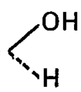 oder ein Sauerstoffatom, und

$R_4$ Wasserstoff, einen Alkanoylrest mit 1 bis 3 Kohlenstoffatomen oder eine Alkylgruppe mit 1 bis 3 Kohlenstoffatomen bedeuten

mit einwertigen Alkanolen mit 1 bis 3 Kohlenstoffatomen oder zweiwertigen Alkanolen mit 2 bis 6 Kohlenstoffatomen unter saurer Katalyse umsetzt.

3. Verfahren nach Anspruch 1, dadurch gekennzeichnet, dass man ein wasserbindendes oder entfernendes Mittel zusetzt.

4. Arzneimittel gekennzeichnet durch einen Gehalt an 3‴-Dehydrocardenolidtridigitoxosiden der Formel (I).

5. Verbindungen gemäss Anspruch 1 zur Verwendung bei der Bekämpfung der Herzinsuffizienz.

## Claims

1. Ketals of 3‴-dehydrocardenolide tridigitoxosides of the Formula (I)

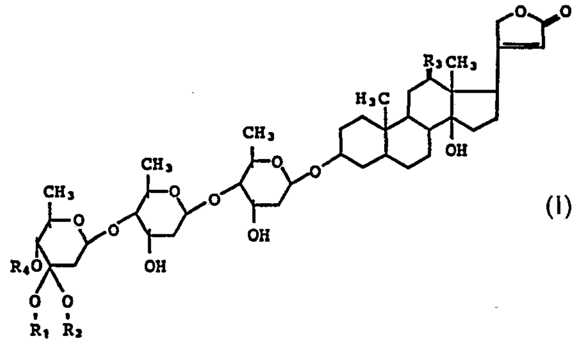

(I)

in which

$R_1$ and $R_2$ represent identical alkyl groups with 1-3 carbon atoms or together a cyclic ketal with 2-6 carbon atoms,

$R_3$ 2 hydrogen atoms, the group 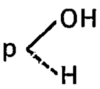 or the radical 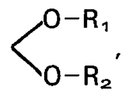, wherein $R_1$ and $R_2$ possess the given meaning,

$R_4$ hydrogen, an alkanoyl radical 1-3 carbon atoms or an alkyl group with 1-3 carbon atoms.

2. Process for the preparation of ketals of 3‴-dehydrocardenolide tridigitoxosides of the Formula (I)

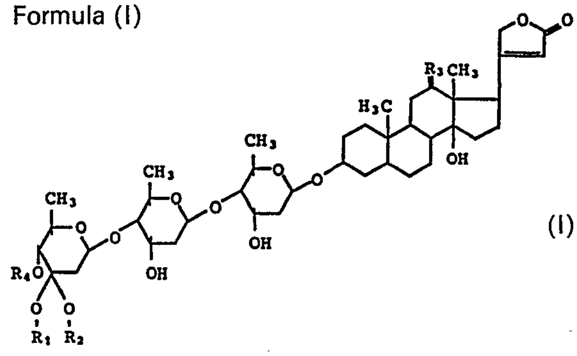

(I)

in which

$R_1$ and $R_2$ represent identical alkyl groups with 1-3 carbon atoms or together a cyclic ketal with 2-6 carbon atoms,

$R_3$ 2 hydrogen atoms, the group 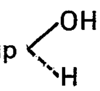 or the radical 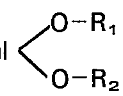, wherein $R_1$ and $R_2$ possess the given meaning,

$R_4$ hydrogen, an alkanoyl radical with 1-3 carbon atoms or an alkyl group with 1-3 carbon atoms,

characterised in that one reacts compounds of the Formula (II)

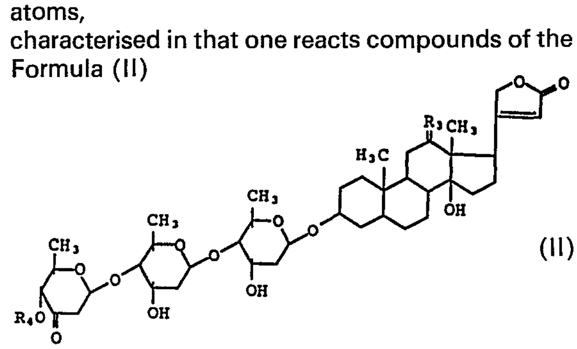

(II)

in which

$R_3$ represents 2 hydrogen atoms, the group 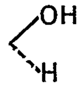 or an oxygen atom, and

$R_4$ hydrogen, an alkanoyl radical with 1-3 carbon atoms or an alkyl group with 1-3 carbon atoms, with monohydroxy alkanols with 1-3 carbon atoms or dihydroxy alkanols with 2-6 carbon atoms with acid catalysts.

3. Process according to Claim 2, characterised in that one adds a water-binding or removing agent.

4. Medicaments characterised by a content of 3‴-dehydrocardenolide tridigitoxoside ketals of Formula (I).

5. Compounds according to Claim 1 for use in the combating of heart insufficiency.

## Revendications

1. Cétals 3‴-déhydrocardénolidetridigitoxosides de formule (I)

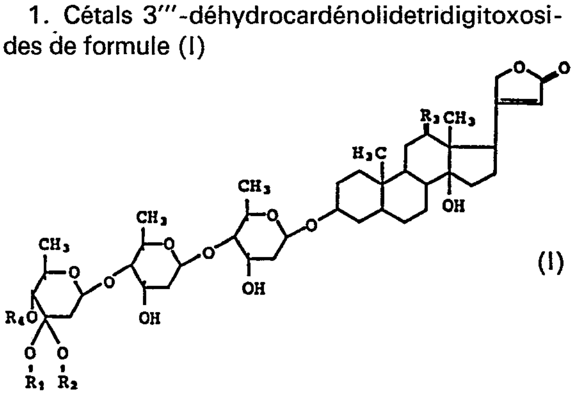

(I)

dans laquelle

$R_1$ et $R_2$ sont des groupes alkyle identiques ayant de 1 à 3 atomes de carbone, ou sont ensemble un cétal cyclique ayant de 2 à 6 atomes de carbone,

$R_3$ représente 2 atomes d'hydrogène, le groupe 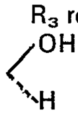 ou le reste 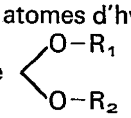 où $R_1$ et $R_2$ ont la signification indiquée,

$R_4$ est de l'hydrogène, un reste alcanoyle ayant de 1 à 3 atomes de carbone ou un groupe alkyle ayant de 1 à 3 atomes de carbone.

2. Procédé de préparation de cétals 3‴-déhydrocardénolidetridigitoxosides de formule (I)

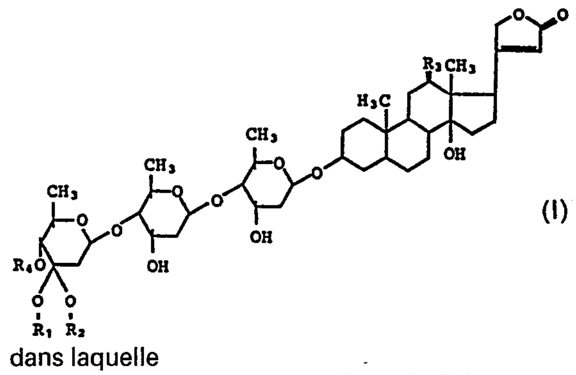

(I)

dans laquelle

$R_1$ et $R_2$ sont des groupes alkyle similaires ayant de 1 à 3 atomes de carbone ou sont ensemble un cétal cyclique ayant de 2 à 6 atomes de carbone,

$R_3$ représente 2 atomes d'hydrogène, le groupe 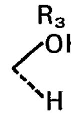 ou le reste 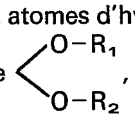, où $R_1$ et $R_2$ ont la signification indiquée,

$R_4$ est de l'hydrogène, un reste alcanoyle ayant de 1 à 3 atomes de carbone ou un groupe alkyle ayant de 1 à 3 atomes de carbone,

caractérisé en ce qu'on fait réagir des composés de formule (II)

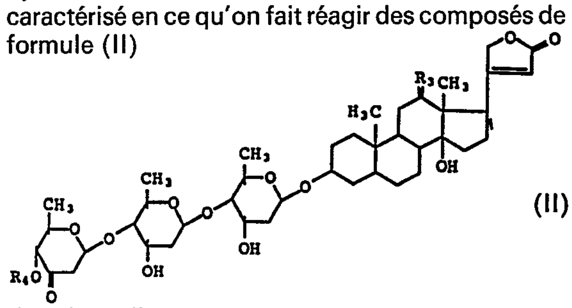

(II)

dans laquelle

$R_3$ représente 2 atomes d'hydrogène, le groupe 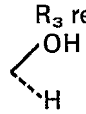 ou un atome d'oxygène, et

$R_4$ est de l'hydrogène, un reste alcanoyle ayant de 1 à 3 atome de carbone ou un groupe alkyle ayant de 1 à 3 atomes de carbone

avec des alcanols monovalents ayant de 1 à 3 atomes de carbone ou avec des alcanols bivalents ayant de 2 à 6 atomes de carbone à l'aide d'un catalyseur acide.

3. Procédé selon la revendication 1, caractérisé en ce qu'on ajoute un agent liant ou éliminant l'eau.

4. Médicament caractérisé en ce qu'il a une teneur en 3‴-déhydrocardénolidetridigitoxosides de formule (I).

5. Composés selon la revendication 1 utilisés pour le traitement de l'insuffisance cardiaque.